(19) Europäisches Patentamt
European Patent Office
Office européen des brevets

(11) Veröffentlichungsnummer: **0 035 768**
**A2**

(12) # EUROPÄISCHE PATENTANMELDUNG

(21) Anmeldenummer: 81101627.8

(22) Anmeldetag: 06.03.81

(51) Int. Cl.³: C 07 C 131/00
C 07 C 59/68, A 01 N 39/02

(30) Priorität: 10.03.80 CH 1861/80

(43) Veröffentlichungstag der Anmeldung:
16.09.81 Patentblatt 81/37

(84) Benannte Vertragsstaaten:
AT BE CH DE FR GB IT LI LU NL SE

(71) Anmelder: F. HOFFMANN-LA ROCHE & CO.
Aktiengesellschaft

CH-4002 Basel(CH)

(72) Erfinder: de Silva, Wijitha, Dr.
Am Sonnenberg
CH-8165 Schöfflisdorf(CH)

(74) Vertreter: Lederer, Franz, Dr. et al,
Patentanwälte Dr. Franz Lederer Reiner F. Meyer
Lucile-Grahn-Strasse 22
D-8000 München 80(DE)

(54) **Oximester, Verfahren und Mittel zu deren Herstellung, ihre Verwendung, sowie diese Oximester enthaltende Mittel.**

(57) Es werden Verbindungen der allgemeinen Formel

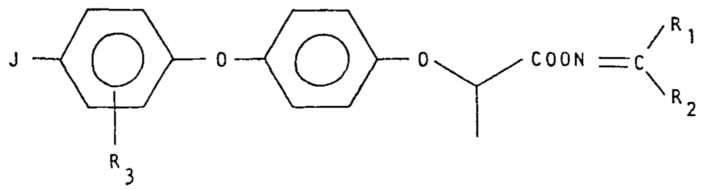

1

worin $R_1$ Wasserstoff, Alkyl mit 1-6 Kohlenstoffatomen oder Cycloalkyl mit 3-6 Kohlenstoffatomen, $R_2$ Wasserstoff, Alkyl mit 1-6 Kohlenstoffatomen, Alkenyl mit 2-6 Kohlenstoffatomen oder Alkinyl mit 2-6 Kohlenstoffatomen, oder $R_1$ und $R_2$ zusammen mit dem Kohlenstoffatom, an das sie geknüpft sind, einen Cyclopentan- oder Cyclohexanring bilden, der gegebenenfalls mit Alkyl mit 1-3 Kohlenstoffatomen mono-, di- oder trisubstituiert sein kann, und $R_3$ Wasserstoff, Halogen oder Nitro darstellt, mit der Massgabe, dass $R_1$ und $R_2$ nicht gleichzeitig Wasserstoff bedeuten, beschrieben, sowie Mittel und Verfahren zu deren Herstellung, sowie ihre Verwendung in Unkrautbekämpfungsmitteln.

EP 0 035 768 A2

F. Hoffmann-La Roche & Co. Aktiengesellschaft, Basel, Schweiz

RAN 6102/25

# Oximester, Verfahren und Mittel zu deren Herstellung, ihre Verwendung, sowie diese Oximester enthaltende Mittel.

Die Erfindung betrifft Oximester der allgemeinen Formel

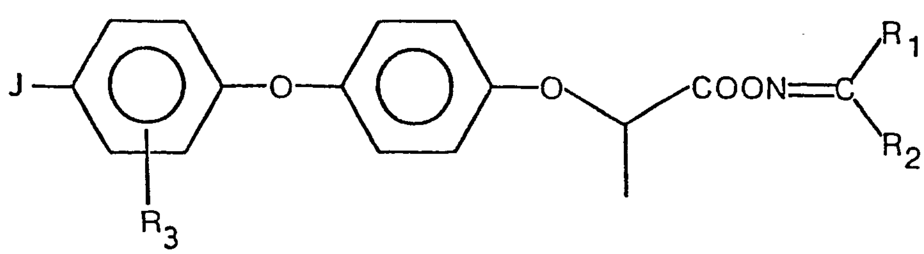

I

worin $R_1$ Wasserstoff, Alkyl mit 1-6 Kohlenstoffatomen oder Cycloalkyl mit 3-6 Kohlenstoffatomen, $R_2$ Wasserstoff, Alkyl mit 1-6 Kohlenstoffatomen, Alkenyl mit 2-6 Kohlenstoffatomen oder

Alkinyl mit 2-6 Kohlenstoffatomen, oder $R_1$ und $R_2$ zusammen mit dem Kohlenstoffatom, an das sie geknüpft sind, einen Cyclopentan- oder Cyclohexanring bilden, der gegebenenfalls mit Alkyl mit 1-3 Kohlenstoffatomen mono-, di- oder trisubstituiert sein kann, und $R_3$ Wasserstoff, Halogen oder Nitro darstellt, mit der Massgabe, dass $R_1$ und $R_2$ nicht gleichzeitig Wasserstoff bedeuten.

Die Erfindung betrifft ferner ein Verfahren zur Herstellung von Verbindungen der Formel I, Vorauflauf- und Nachauflauf-herbicide Mittel, Verfahren zur Herstellung solcher Mittel und zu deren Verwendung, sowie die Verwendung derartiger herbicider Mittel an sich.

Das Verfahren zur Herstellung von Verbindungen der Formel I ist dadurch gekennzeichnet, dass man

a) eine Säure der Formel

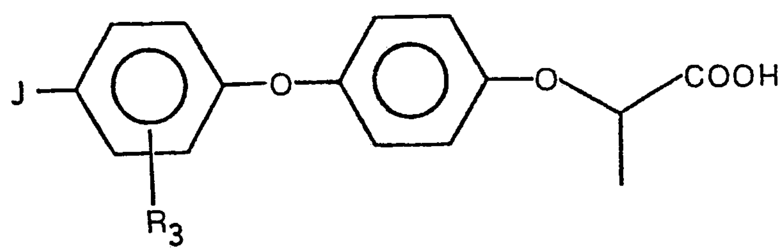 II

worin $R_3$ die für Formel I angegebene Bedeutung besitzt,

oder ein reaktionsfähiges Derivat einer solchen Säure, mit einem Oxim

$$R_1R_2CNOH \qquad III$$

worin $R_1$ und $R_2$ die für Formel I angegebene Bedeutung besitzen,

umsetzt, oder

b) eine Verbindung der allgemeinen Formel

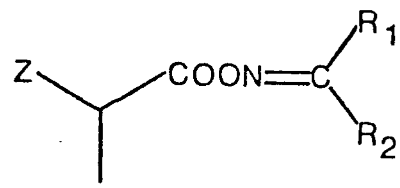

IV

worin Z Chlor, Brom, Jod, Mesyloxy oder Tosyloxy bedeutet und $R_1$ und $R_2$ die für Formel I angegebene Bedeutung besitzen,

mit einer Verbindung der allgemeinen Formel

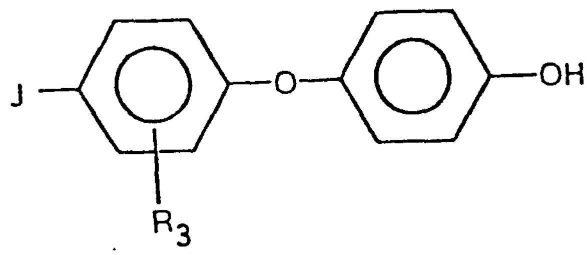

V

worin $R_3$ die oben angegebene Bedeutung hat,

oder einem Alkalimetallsalz davon, erforderlichenfalls in Gegenwart einer Base, umsetzt.

Der Ausdruck "Alkyl" mit 1-6 bzw. 1-3 Kohlenstoffatomen umfasst - wenn nicht anderweitig angegeben - sowohl geradkettige als auch verzweigte Kohlenwasserstoffradikale mit 1-6 bzw. 1-3 Kohlenstoffatomen, wie Methyl, Aethyl, Propyl, lsopropyl, Butyl, Isobutyl, tert.-Butyl und dgl.

Die Ausdrücke "Alkenyl" und "Alkinyl" mit 2-6 Kohlenstoffatomen umfassen ungesättigte geradkettige und verzweigte Kohlenwasserstoffradikale mit bis zu 6 Kohlenstoffatomen wie Allyl, Butenyl, Isobutenyl, Pentenyl, Isopentenyl und dgl. sowie Propargyl, Butinyl, Isobutinyl und dgl.

Unter "Halogen" sind Fluor, Chlor, Brom und Jod zu verstehen, wobei Chlor und Jod bevorzugt sind.

Bevorzugte Verbindungen der Formel I sind solche, worin $R_1$ und $R_2$ Alkylreste mit 1-2 Kohlenstoffatomen, insbesondere Methyl, und $R_3$ Wasserstoff oder Chlor bedeuten.

Besonders bevorzugte Verbindungen der Formel I sind das Aceton-O-/2-[p-(p-jodphenoxy)-phenoxy]propionyl/-oxim und das Cyclopropylmethylketon-O-/2-[p-(p-jodphenoxy)phenoxy]-propionyl/-oxim, Aceton-O-/2-[p-(2-chlor-4-jodphenoxy)-phenoxy]propionyl/-oxim, insbesondere die D-Isomeren dieser Verbindungen.

Weitere interessante Verbindungen der Formel I sind:

2-Butanon-O-/2-[p-(p-jodphenoxy)phenoxy]propionyl/-oxim; 3-Pentanon-O-/2-[p-(p-jodphenoxy)phenoxy]propionyl/-oxim; 3-Heptanon-O-/2-[p-(p-jodphenoxy)phenoxy]propionyl/-oxim; (2-Methyl-2-penten-4-on)-O-/2-[p-(p-jodphenoxy)-phenoxy]propionyl/-oxim; Diisopropylketon-O-/2-[p-(p-jodphenoxy)phenoxy]propionyl/-oxim; 6-Undecanon-O-/2-[p-(p-jodphenoxy)phenoxy]propionyl/-oxim; Isopropyläthylketon-O-/2-[p-(p-jodphenoxy)phenoxy]propionyl/-oxim; Isobutyläthylketon-O-/2-[p-(p-jodphenoxy)phenoxy]propionyl/-oxim; Cyclopentanon-O-[p-(p-jodphenoxy)phenoxy]propionyl/-oxim; Cyclohexanon-O-/2-[p-(p-jodphenoxy)phenoxy]propionyl/-oxim; 2,4,4-Trimethylcyclohexanon-O-/2-[p-(p-jodphenoxy)-phenoxy]propionyl/-oxim, insbesondere die entsprechenden D-Isomeren.

In einer Ausführungsform des Verfahrens zur Herstellung von substituierten Phenoxyalkancarbonsäureoximestern der allgemeinen Formel I wird eine Säure der Formel II oder ein reaktionsfähiges Derivat hiervon mit einem Oxim der Formel III umgesetzt.

Der Ausdruck "reaktionsfähiges Derivat der Säure" bedeutet vor allem ein Säurehalogenid, insbesondere das Chlorid, oder ein Säureanhydrid.

Zur Herstellung der substituierten Phenoxyalkancarbonsäureoximester der allgemeinen Formel I wird die Veresterung der Säure der allgemeinen Formel II oder des Säurederivates mit dem Oxim der allgemeinen Formel III vorzugsweise in einem geeigneten inerten Lösungsmittel, bei Temperaturen von etwa -10 bis +100°C durchgeführt. Besonders bevorzugt ist +20 bis +70°C. Bei Verwendung eines Säurehalogenids als reaktionsfähiges Derivat der Säure wird die Umsetzung mit dem Oxim zweckmässig bei Raumtemperatur und in Gegenwart eines Säureacceptors, z.B. eines tertiären Amins, wie Pyridin oder Triäthylamin oder in alkalischer Lösung nach Schotten-Baumann durchgeführt. Man erhält in hoher Ausbeute den entsprechenden Ester. Als Säurehalogenide werden die Säurechloride bevorzugt. Die Umsetzung wird vorzugsweise in Gegenwart eines inerten Lösungsmittels durchgeführt, wie Benzol, Toluol, Petroläther oder nach Schotten-Baumann in alkalischer Lösung. Bei Verwendung des Anhydrides der Säure der allgemeinen Formel II kann der entsprechende Oximester der allgemeinen Formel I in hoher Ausbeute durch Erhitzen des Anhydrides mit dem Oxim der allgemeinen Formel III in Gegenwart einer Base, vorzugsweise einem Alkalimetallcarbonat hergestellt werden. Besonders bevorzugt ist Natriumcarbonat.

Wenn als Ausgangsmaterial die freie Säure der Formel II verwendet wird, so wird diese mit dem Oxim der Formel III in Gegenwart von Dicyclohexylcarbodiimid umgesetzt. Zu diesem Zweck wird die Säure der Formel II in einem inerten organischen Lösungsmittel wie einem chlorierten Kohlenwasserstoff, beispielsweise Dichlormethan, Chloroform, Tetrachlorkohlenstoff oder Trichloräthan, einem Aether, wie beispielsweise Diäthyläther, Diisopropyläther oder Dioxan, einem aromatischen Kohlenwasserstoff, beispielsweise Benzol, Toluol oder Xylol usw. gelöst und danach wird das Oxim der Formel III in diesem Gemisch suspendiert. Das Dicyclohexylcarbodiimid wird zweckmässig im selben Lösungsmittel gelöst und zum Reaktionsgemisch zugegeben. Die Umsetzung kann in einem Temperaturbereich zwischen 0° und der Siedetemperatur des Reaktionsgemisches erfolgen, vorzugsweise zwischen Raumtemperatur und 50°C. Nach etwa 2 Stunden ist die Umsetzung beendet, das Reaktionsgemisch wird filtriert und danach eingedampft. Der Rückstand wird gegebenenfalls zur Reinigung umkristallisiert oder chromatographiert.

In einer weiteren Ausführungsform zur Herstellung von Verbindungen der Formel I wird eine Verbindung der Formel IV mit einer Verbindung der Formel V oder einem Alkalimetallsalz, insbesondere dem Natrium- oder Kaliumsalz, in an sich bekannter Weise umgesetzt. Die Umsetzung erfolgt zweckmässigerweise in einem inerten organischen Lösungsmittel wie Kohlenwasserstoffen, z.B. Benzol oder Toluol, Aethern, z.B. Diäthyläther, Tetrahydrofuran, Dimethoxyäthan oder Hexamethylphosphorsäuretriamid, und dgl. Temperatur und Druck sind nicht kritisch, und man arbeitet bevorzugt bei einer Temperatur zwischen -20°C und der Rückflusstemperatur der Reaktionsmischung, vorzugsweise zwischen -10° und 30°C.

Die Verbindungen der allgemeinen Formel I schliessen auch optische Isomere ein, da sie ein asymmetrisches Kohlenstoffatom in der α-Stellung besitzen. Weitere asymmetrische Kohlenstoffatome können die Esterkomponenten enthalten. Erwünschtenfalls können die racemischen Verbindungen unter Verwendung bekannter Verfahren in rechtsdrehende und linksdrehende Verbindungen getrennt werden. Derartige Verfahren sind beispielsweise in Industrial and Engineering Chemistry 60 (8) 12-28 beschrieben. Die Isomeren und die racemischen Mischungen besitzen alle herbicide Aktivität, jedoch ist das Ausmass dieser Aktivität unterschiedlich. Am grössten ist die Aktivität beim D-Isomeren, danach folgt die racemische Mischung und dann das L-Isomere. Es wurde im Zusammenhang mit derartigen Untersuchungen gefunden, dass beispielsweise in bestimmten Versuchsanordnungen das D-Isomere des Aceton-O-/2-[p-(p-jodphenoxy)phenoxy]-propionyl/-oxim eine grössere Aktivität als die racemische Mischung aufweist. Die D-Isomeren der Verbindungen der Formel I sind daher bevorzugt.

Die Isomeren - insbesondere die D-Isomeren - können auch durch Synthese aus entsprechenden optisch aktiven Ausgangsmaterialien hergestellt werden.

Infolge der Stickstoff-Kohlenstoff-Doppelbindung in der

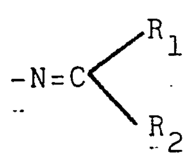

Gruppe erhält man jeweils zwei geometrische Isomere (wenn $R_1$ und $R_2$ unterschiedliche Bedeutung haben), die als syn- und anti-Form bezeichnet werden. Es gelingt in gewissen Fällen, derartige Isomere zu isolieren. Diese sind ebenfalls Gegenstand der Erfindung.

Die vorliegende Erfindung betrifft auch herbicide Zusammensetzungen, die als aktiven Bestandteil eine oder mehrere Verbindungen der Formel I, wie oben definiert, enthalten. Die herbicide Zusammensetzung enthält zweckmässigerweise zumindest eines der folgenden Materialien: Trägerstoffe, Netzmittel, inerte Verdünnungsmittel und Lösungsmittel.

Die erfindungsgemässen Verbindungen der Formel I zeichnen sich gegenüber bekannten Verbindungen ähnlicher Struktur, bei im wesentlichen gleicher herbicider Wirkung, durch wesentlich bessere Verträglichkeit gegenüber Nutzpflanzen, z.B. Weizen oder Zuckerrüben, aus.

Die erfindungsgemässen Vorauflauf- und Nachauflauf-Herbicide eignen sich besonders zur Bekämpfung von Ungräsern, insbesondere von Ackerfuchsschwanz (Alopecurus myosuroides) und Hirsearten wie beispielsweise Hühnerhirse (Echinochloa crus-galli), grosse Borstenhirse (Setaria faberii) und haarartige Hirse (Panicum capillare in Getreide - insbesondere Gerste-, Hafer- und Weizen- sowie Reis-, Baumwolle-, Soya-, Zuckerrüben und Gemüsekulturen. Besonders bevorzugt eignen sich die erfindungsgemässen Vorauflauf- und Nachauflauf-Herbicide zur Bekämpfung von Ungräsern in Zuckerrübenkulturen. So besitzt beispielsweise das Aceton-O-/2-[p-(p-jodphenoxy)phenoxy]propionyl/-oxim bei einer Konzentration von 1,25 kg/ha genügend Wirksamkeit gegen Ungräser, ohne jedoch die Zuckerrübenkulturen zu schädigen. Im allgemeinen genügt eine Konzentration von 0,3-2 kg/ha, vorzugsweise 0,5-1,0 kg/ha, besonders bevorzugt 0,8 kg/ha, um den gewünschten herbiciden Effekt mit Verbindungen der Formel I zu erzielen.

Die Verbindungen der Formel I sind im allgemeinen wasserunlöslich und können nach irgendeiner der für unlösliche Verbindungen üblichen Methoden konfektioniert werden.

Wenn gewünscht, können die Verbindungen der Formel I in einem mit Wasser nicht mischbaren Lösungsmittel, wie beispielsweise einem hochsiedenden Kohlenwasserstoff, gelöst werden, das zweckmässigerweise gelöste Emulgatoren

enthält, so dass es bei Zugabe von Wasser als selbstemulgierbares Oel wirkt.

Die Verbindungen der Formel I können auch mit einem Netzmittel mit oder ohne inertes Verdünnungsmittel zur Bildung eines netzbaren Pulvers vermischt werden, welches in Wasser löslich oder dispergierbar ist, oder sie können mit dem inerten Verdünnungsmittel zur Bildung eines festen oder pulverförmigen Produktes vermischt werden.

Inerte Verdünnungsmittel, mit welchen die Verbindungen der Formel I verarbeitet werden können, sind feste inerte Medien, einschliesslich pulverförmige oder feinverteilte Feststoffe, wie beispielsweise Tone, Sande, Talk, Glimmer, Düngemittel und dgl., wobei solche Produkte entweder staubförmig oder als Materialien mit grösserer Teilchengrösse vorliegen können.

Die Netzmittel können anionische Verbindungen, wie beispielsweise Seifen, Fettsulfatester, wie Dodecylnatriumsulfat, Octadecylnatriumsulfat und Cetylnatriumsulfat, fettaromatische Sulfonate, wie Alkylbenzolsulfonate oder Butylnaphthalinsulfonate, komplexere Fettsulfonate, wie die Amidkondensationsprodukte von Oelsäure und N-Methyltaurin oder das Natriumsulfonat von Dioctylsuccinat sein.

Die Netzmittel können auch nichtionische Netzmittel, wie beispielsweise Kondensationsprodukte von Fettsäuren, Fettalkoholen oder fettsubstituierten Phenolen mit Aethylenoxyd, oder Fettsäureester und -äther von Zuckern oder mehrwertigen Alkoholen oder die Produkte, die aus letzteren durch Kondensation mit Aethylenoxyd erhalten werden, oder die Produkte, die als Blockpolymere von Aethylenoxyd und Propylenoxyd bekannt sind, sein. Die Netzmittel können auch kationische Mittel, wie beispielsweise Cetyltrimethylammoniumbromid und dgl., sein.

Die herbicide Zusammensetzung kann auch in Form einer Aerosolverbindung vorliegen, wobei zweckmässigerweise zusätzlich zum Treibgas, welches geeigneterweise ein polyhalogeniertes Alkan, wie Dichlordifluormethan ist, ein Co-Solvens und ein Netzmittel verwendet wird.

Die herbiciden Zusammensetzungen gemäss der vorliegenden Erfindung können zusätzlich zu den Verbindungen der Formel I, Synergisten und andere aktive Insekticide, Bakterecide, Herbicide und Fungicide enthalten.

In ihren verschiedenen Anwendungsgebieten können die erfindungsgemässen Verbindungen in unterschiedlichen Mengenverhältnissen eingesetzt werden.

Die erfindungsgemässen Unkrautbekämpfungsmittel können in einer Form vorliegen, die sich für die Lagerung und den Transport eignen. Solche Formen können z.B. 2-90% an einem oder mehreren Wirkstoffen der Formel I enthalten. Diese Formen können dann mit gleichen oder verschiedenen Trägermaterialien bis zu Konzentrationen verdünnt werden, die sich für den praktischen Gebrauch eignen. Im gebrauchsfertigen Mittel können dann Wirkstoffkonzentrationen von 2-80% (Gewichtsprozent) vorliegen. Die Wirkstoffkonzentration kann jedoch auch kleiner oder grösser sein. Je nach Verwendungszweck ist eine Wirkstoffkonzentration von 2-8% bzw. 25-50% besonders bevorzugt.

Wertvolle Ausgangsmaterialien zur Herstellung der Verbindungen der Formel I sind Verbindungen der allgemeinen Formel

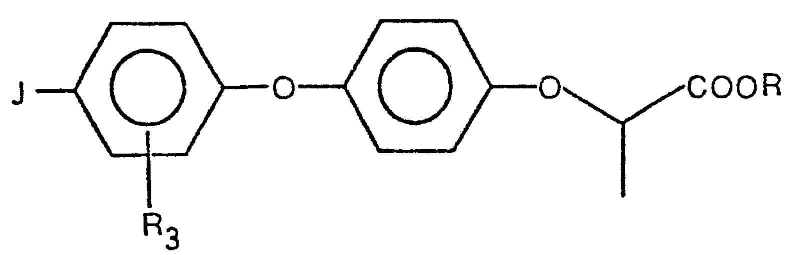

IIa

worin R Wasserstoff oder Alkyl mit 1-6 Kohlenstoffatomen und $R_3$ Wasserstoff, Halogen oder Nitro bedeuten.

Die Formel IIa umfasst somit die Säuren der Formel II und ausserdem die entsprechenden $C_1$-$C_6$ Alkylester, welche durch Verseifung in die freien Säuren übergeführt werden können.

Besonders bevorzugte Verbindungen der Formel IIa sind die 2-[p-(p-Jodphenoxy)phenoxy]-propionsäure und der 2-(p-Jodphenoxy)phenoxy]-propionsäureäthylester, die 2-[p-(2-Chlor-4-jodphenoxy)phenoxy]-propionsäure und der 2-[p-(2-Chlor-4-jodphenoxy)phenoxy]-propionsäure-äthylester, insbesondere die D-Isomeren dieser Verbindungen.

Die vorstehend angegebenen Ausgangsmaterialien der Formel IIa sind in Form ihrer D-Isomeren neue Verbindungen. Sie sind zusätzlich auch als Herbicide wertvoll, da sie ein ähnliches Aktivitätsspektrum wie die Verbindungen der Formel I aufweisen. Sie besitzen den Vorteil, dass sie im Vergleich zum jeweiligen Racemat beispielsweise eine geringere Phytotoxizität auf Baumwolle und Soyabohnen aufweisen. Diese neuen D-Isomeren der Formel IIa bilden ebenfalls einen Gegenstand der vorliegenden Erfindung.

Die Ausgangsmaterialien der Formeln V und II können gemäss dem folgenden Reaktionsschema hergestellt werden:

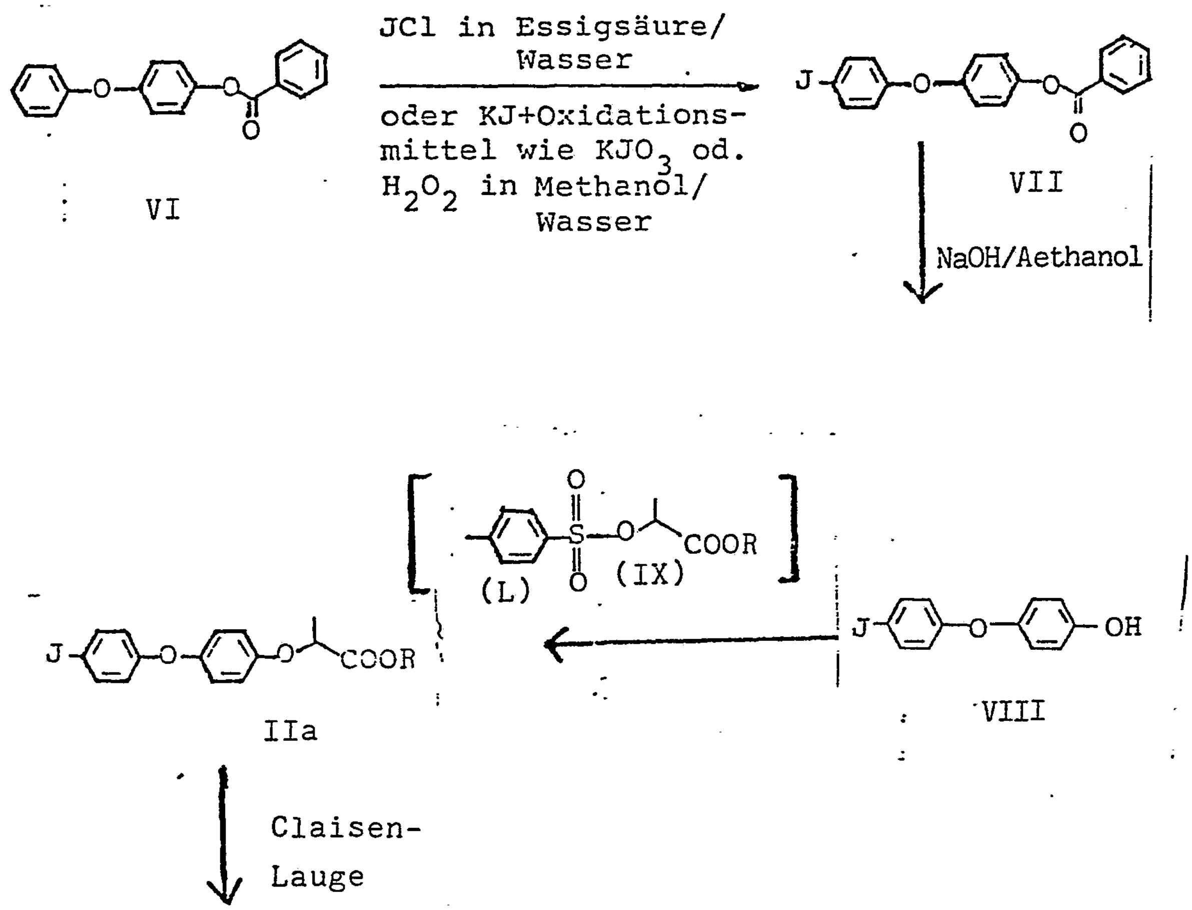


VI

VII

(L) (IX)

VIII

IIa

II

Hierbei wird das p-Phenoxyphenylbenzoat der Formel VI entweder mit Jodmonochlorid in wässriger Essigsäure umgesetzt oder mit Kaliumjodid und einem Oxidationsmittel, beispielsweise Kaliumjodat oder Wasserstoffperoxid in wässrigem Methanol, behandelt, wobei man das Jodderivat der Formel VII erhält. Dieses wird durch Erhitzen in Lauge (z.B. alkoholischem Natriumhydroxid) zum p-(p-Jodphenoxy)-phenol VIII verseift.

Durch Behandlung der Verbindung VIII bzw. eines entsprechenden Alkalimetallphenolats mit einem Milchsäurealkylester-tolylsulfonat, beispielsweise dem entsprechenden Aethylester (R = Aethyl), gelangt man über die intermediär gebildete Verbindung der Formel IX zum Ester IIa, der durch Behandlung

mit Claisen-Lauge zur freien Säure II verseift werden kann. Wenn man hierbei ein optisch aktives Milchsäurealkylester-tolylsulfonat verwendet, so erhält man einen optisch aktiven Ester IIa und eine optisch aktive Säure II. So ergibt L-(-)-Milchsäureäthylester-tolylsulfonat den D(+)-Aethylester IIa und die D(+)-Säure II.

Die folgenden Beispiele sollen die vorliegende Erfindung illustrieren. Alle Temperaturen sind in °C angegeben.

Beispiel 1

1,1 g einer 50%-igen Suspension von Natriumhydrid in Mineralöl werden in einer Stickstoffatmosphäre zweimal mit je 5,0 ml Tetrahydrofuran gewaschen, dann in 15,0 ml Tetrahydrofuran eingetragen und tropfenweise mit einer Lösung von 7 g p-(p-Jodphenoxy)phenol, gelöst in 30,0 ml Dimethylformamid, versetzt.

In das Gemisch werden anschliessend 5,2 g 2-Brompropionylacetonoxim in 20,0 ml Dimethylformamid eingetropft.

Das Reaktionsgemisch wird 2 Stunden unter Rückflussbedingungen erhitzt, danach abgekühlt, auf Eis gegossen und erschöpfend mit Aether extrahiert. Der Aetherextrakt wird mit Wasser gewaschen, über Natriumsulfat getrocknet und unter vermindertem Druck eingedampft.

Das zurückbleibende Aceton-O-/2-[p-(p-jodphenoxy)-phenoxy]-propionyl/-oxim kann durch Adsorption an Kieselgel gereinigt werden; $n_D^{20}$: 1,5887.

Das hierbei als Ausgangsmaterial verwendete p-(p-Jodphenoxy)-phenol kann wie folgt erhalten werden:

Eine Lösung von 290,4 g p-Phenoxyphenylbenzoat in 1400 ml Eisessig wird unter Rühren zum Sieden erhitzt. Dazu tropft man innerhalb 10 Minuten eine Lösung von 340 g Jodmonochlorid in 800 ml Eisessig. Danach fügt man 1800 ml kochendes Wasser zu und rührt bei 95 - 100°C 2 Stunden weiter. Schliesslich wird nach abermaligem Versetzen mit 1800 ml kochendem Wasser auf 20°C abgekühlt, wobei p-(p-Jodphenoxy)-phenylbenzoat auskristallisiert.

Nach Zugabe von 1000 ml einer 10%-igen Natriumhydrogensulfitlösung wird der Kristallbrei abgenutscht

und mit 1000 ml verdünnter Essigsäure und 2000 ml entsalztem Wasser nachgewaschen. Das Produkt wird aus Essigester-n-Hexan (1:4) umkristallisiert.
Fp: 121-124°C.

300 g des so erhaltenen p-(p-Jodphenoxy)-phenylbenzoats werden in 2800 ml 96%-igem Aethanol suspendiert, mit 700 g 20%-iger Natriumhydroxydlösung versetzt und 90 Minuten auf dem siedenden Wasserbad erhitzt. Anschliessend wird der Alkohol als azeotropisches Gemisch abdestilliert und der Rückstand mit 1500 ml entsalztem Wasser versetzt. Nach Zugabe von 380 ml konzentrierter Salzsäure wird 3 mal mit je 1500 ml Dichlormethan extrahiert.

Der Methylenchloridextrakt wird je 2 mal mit 500 ml gesättigter Natriumhydrogencarbonatlösung, 2n Salzsäure und entsalztem Wasser gewaschen, über Natriumsulfat getrocknet und bis auf 500 ml unter vermindertem Druck eingedampft. Nach Zugabe von 500 ml heissem n-Hexan erhält man 201.g g p-(p-Jodphenoxy)-phenol;
Fp: 119-121°C.

## Beispiel 2

21 In zu Beispiel 1 analoger Weise erhält man durch Umsetzung von 2-Brompropionylcyclopropylmethylketonoxim mit p-(p-Jodphenoxy)-phenol das Cyclopropylmethylketon-O-/2-[p-(p-jodphenoxy)phenoxy]-propionyl/-oxim;
$n_D^{20}$: 1.588.

Beispiel 3

4 g D-2-[p-(p-Jodphenoxy)phenoxy]propionsäure und 0,76 g Acetonoxim werden in 40 ml Methylenchlorid gelöst. Dazu tropft man bei Raumtemperatur eine Lösung von 2,14 g Dicyclohexylcarbodiimid in 15 ml Methylenchlorid innerhalb 5 Minuten zu. Es wird 1 Stunde bei Raumtemperatur weitergerührt und der ausgefallene N,N'-Dicyclohexylharnstoff abfiltriert.

Das Filtrat wird auf Wasser gegossen und mit 200 ml Methylenchlorid extrahiert. Der Extrakt wird über Natriumsulfat getrocknet und unter vermindertem Druck eingedampft. Das zurückbleibende Aceton-O-/2-D-[p-(p-jodphenoxy)phenoxy] propionyl/-oxim kann durch Adsorption an der 10-fachen Menge Kieselgel gereinigt werden.

Ausbeute: 73,2% $n_D^{20}$: 1,5885 $\alpha_D$: +35,44° (C=1,97% in $CHCl_3$)

Die hierbei als Ausgangsmaterial verwendete D-2-[p-(p-Jodphenoxy)phenoxy]propionsäure kann wie folgt erhalten werden:

Zu 134,6 g Natrium-p-(p-jodphenoxy)phenolat in 500 ml Dimethylformamid tropft man bei Raumtemperatur 109,1 g L-(-)-Milchsäureäthylester-tolylsulfonat, gelöst in 300 ml Dimethylformamid zu. Es wird eine Stunde weitergerührt und auf 1000 ml Wasser gegossen. Das Produkt wird mit 2 x 500 ml Aethylacetat extrahiert. Die organische Phase wird nochmals mit 500 ml entsalztem Wasser gewaschen, über Natriumsulfat getrocknet und unter vermindertem Druck eingedampft. Der Rückstand kristallisiert aus n-Hexan bei -20°. Man erhält 127,1 g D(+)-2-[p-(p-Jodphenoxy)phenoxy]-propionsäureäthylester.

Fp: 48-51°C; $\alpha_D^{22}$: 16,88 (c=1,84% in $CHCl_3$)

121 g des so erhaltenen Aethylesters werden in 200 ml Methanol gelöst. Dazu gibt man rasch 71,4 ml Claisen-Lauge zu. Die Temperatur steigt dabei auf 40°. Es wird eine Stunde bei Raumtemperatur gerührt, anschliessend auf 1000 g Eis gegossen und mit 300 ml 2n Salzsäure angesäuert. Das Produkt wird mit 1000 ml Aethylacetat extrahiert. Die organische Phase wird mit 3 x 500 ml entsalztem Wasser neutralgewaschen, über Natriumsulfat getrocknet und unter vermindertem Druck abdestilliert.

Man erhält nach dem Umkristallisieren aus Methylenchlorid/n-Hexan (1:2) 93,6 g D(+)-2-[p-(p-Jodphenoxy)phenoxy]-propionsäure.
Fp: 121-124°C; $\alpha_D^{22}$: 13,03 (c = 1,82% in $CHCl_3$)

Beispiel 4

5,0 g D-2-[p-(2-Chlor-4-jodphenoxy)phenoxy]-propionsäure und 0,9 g Acetonoxim werden in 70,0 ml Dichlormethan vorgelegt. Nun wird unter Rühren eine Lösung von 2,5 g N,N-Dicyclohexylcarbodiimid in 30,0 ml Dichlormethan zugesetzt und 1 Stunde weiter gerührt. Hierauf wird filtriert und mit 10 ml Aether nachgewaschen. Das Lösungsmittel wird abgedampft und der Rückstand über die 10-fache Menge Kieselgel filtriert (Laufmittel: Hexan/Aethylacetat, 9:1). Man erhält Aceton-O-/2-D-[p-(2-chlor-4-jodphenoxy)phenoxy]propionyl/-oxim; $[\alpha]_D^{20}$ = +34,97°C (c = 1,38% in Chloroform).

Die hierbei als Ausgangsmaterial verwendete D-2-[p-(2-Chlor-4-jodphenoxy)phenoxy]-propionsäure kann wie folgt erhalten werden:

In einem 2,5-Liter Sulfierkolben mit Rührer, Thermometer, Tropftrichter und Kühler werden 269,5 g Hydrochinon und 90,6 g Calciumhydroxid in 1224 ml Dimethylsulfoxid vorgelegt. Dann wird unter Rühren eine Lösung von 235,0 g 3,4-Dichlornitrobenzol in 367 ml Dimethylsulfoxid zugegeben und

bei 80°C weitergerührt. Hierauf wird mit 800 ml Salzsäure 2N bis pH 1 angesäuert und dreimal mit je 800 ml Aethylacetat extrahiert. Die Aethylacetatphase wird mit 800 ml Natriumhydrogencarbonat und 800 ml Wasser nachgewaschen. Nach dem Trocknen über Natriumsulfat wird das Lösungsmittel eingedampft. Der Eindampfrückstand wird aus Alkohol/Wasser (1:1) umkristallisiert. Man erhält p-(2-Chlor-4-nitrophenoxy)-phenol mit einem Schmelzpunkt von 152°C.

In einem 1,5-Liter Sulfierkolben werden 110 g des so erhaltenen p-(2-Chlor-4-nitrophenoxy)-phenols zusammen mit 9,4 g Aktivkohle und 5,4 g Eisen-III-chlorid in 680 ml Methanol zum Rückfluss erhitzt. Innerhalb von 30 Minuten werden nun unter Rühren 33,0 g Hydrazinhydrat zugetropft. Es wird 20 Stunden bei Rückfluss weitergerührt, hierauf abgekühlt, filtriert und das Lösungsmittel abgedampft. Der Rückstand wird in 400 ml Aethylacetat aufgenommen, ungelöste Teile werden abfiltriert und das Phenol mit 400 ml n-Hexan gefällt und das Produkt auskristallisiert. Man erhält p-(4-Amino-2-chlorphenoxy)phenol.

In einem 250 ml Sulfierkolben werden 5,4 g Jod und 6,0 g Isopentylnitrit in 100 ml Acetonitril vorgelegt und das Gemisch auf 65°C erhitzt. Nun wird eine Lösung von 10,0 g p-(4-Amino-2-chlorphenoxy)phenol in 50 ml Acetonitril langsam, unter Rühren zugetropft. Hierauf wird bei 65°C 20 Minuten weitergerührt. Das Reaktionsgemisch wird dann auf 200 g Eis gegossen, dreimal mit je 200 ml Aethylacetat extrahiert, zweimal mit 200 ml gesättigter Natriumthiosulfat-Lösung und zweimal mit 200 ml Wasser gewaschen. Nach dem Trocknen über Natriumsulfat wird das Lösungsmittel abgedampft. Der Rückstand wird über die 10-fache Menge Kieselgel filtriert (Laufmittel: Hexan/Aethylacetat, 9:1).

12 g des so erhaltenen p-(2-Chlor-4-jodphenoxy)phenol werden in 100 ml Dimethylformamid gelöst. Unter Kühlen werden 0,83 g Natriumhydrid zugegeben. Nach beendeter Wasserstoff-Entwicklung wird eine Lösung von L-(-)-Milchsäure-äthylester-tolylsulfinat in 50 ml Dimethylformamid langsam zugetropft und es wird 1 Stunde bei Raumtemperatur gerührt. Hierauf wird das Reaktionsgemisch auf 500 ml Wasser gegossen, dreimal mit je 500 ml Aethylacetat extrahiert, zweimal mit je 150 ml Natronlauge 2N, und mit 600 ml Wasser neutral gewaschen. Nach dem Trocknen über Natriumsulfat wird das Lösungsmittel abgedampft. Der erhaltene Rückstand wird über die 10-fache Menge Kieselgel filtriert (Laufmittel: Hexan; am Schluss Hexan/Aethylacetat 9:1). Man erhält den 2-D-[p-(2-Chlor-4-jodphenoxy)phenoxy]-propionsäure-äthylester; $[\alpha]_D^{20}$ +15,03° (c = 1,57% in $CHCl_3$).

In einem 200 ml Sulfierkolben werden 8,8 g des so erhaltenen 2-D-[p-(2-Chlor-4-phenoxy)phenoxy]-propionsäure-äthylesters und 6 ml Claisenlauge in 100 ml Methanol unter Rühren zum Rückfluss erhitzt; hierauf wird 1 Stunde weitergerührt. Das Reaktionsgemisch wird dann mit 100 ml Salzsäure 2N bis pH 1 angesäuert, dreimal mit je 150 ml Aethylacetat extrahiert und mit 600 ml Wasser neutral gewaschen. Nach dem Trocknen über Natriumsulfat wird das Lösungsmittel abgedampft. Der erhaltene Rückstand wird über die 10-fache Menge Kieselgel filtriert. (Laufmittel: Hexan/Aethylacetat 7:3). Man erhält die 2-D-[p-(2-Chlor-4-jodphenoxy)phenoxy]-propionsäure; $[\alpha]_D^{20}$ +8,89° (c = 1,61% in Chloroform).

## Beispiel 5

In zu Beispiel 3 analoger Weise erhält man durch Umsetzung von D-2-[p-(p-Jodphenoxy)phenoxy]propionsäure mit Cyclopropylmethylketonoxim das Cyclopropylmethylketon-O-/2-D-[p-(p-jodphenoxy)phenoxy]propionyl/-oxim; $n_D^{20}$: 1,5866; $\alpha_D$: +43,05 (c = 1,56% in $CHCl_3$).

Beispiel 6

In zu den Beispielen 1-4 analoger Weise können die folgenden Verbindungen erhalten werden:

2-Butanon-O-/2-D-[p-(p-jodphenoxy)phenoxy]propionyl/-oxim; $\alpha_D^{20}$ = +46,6°, $n_D^{20}$ = 1,5832

4-Heptanon-O-/2-D-[p-(p-jodphenoxy)phenoxy]propionyl/-oxim; $\alpha_D^{22}$ = +50,25°, $n_D^{20}$ = 1,5666

(2-Methyl-2-penten-4-on)-O-D-[p-(p-jodphenoxy)-phenoxy]-propionyl/-oxim; $\alpha_D^{22}$ = +46,96°, $n_D^{20}$ = 1,5880

Diisopropylketon-O-/2-D-[p-(p-jodphenoxy)phenoxy]-propionyl/-oxim; $\alpha_D^{22}$ = +44,1°, $n_D^{20}$ = 1,5660

6-Undecanon-O-/2-D-[p-(p-jodphenoxy)phenoxy]propionyl/-oxim; $\alpha_D^{22}$ = +48,09°, $n_D^{20}$ = 1,5496

Isopropyläthylketon-O-/2-D-[p-(p-jodphenoxy)phenoxy]-propionyl/-oxim; $\alpha_D^{22}$ = +51,46°, $n_D^{20}$ = 1,5703

Isobutyläthylketon-O-/2-D-[p-(p-jodphenoxy)phenoxy]-propionyl/-oxim; $\alpha_D^{22}$ = +50,64°, $n_D^{20}$ = 1,5652

Cyclopentanon-O-/2-D-[p-(p-jodphenoxy)phenoxy]propionyl/-oxim; $\alpha_D^{22}$ = +46,2°

Cyclohexanon-O-/2-D-[p-(p-jodphenoxy)phenoxy]propionyl/-oxim; $\alpha_D^{20}$ = +49,4°, $n_D^{20}$ = 1,5802

4-Methyl-3-pentanon-O-/2-D-[p-(p-jodphenoxy)phenoxy]-propionyl/-oxim; $\alpha_D^{20}$ = +51,46°, $n_D^{20}$ = 1,5703

5-Methyl-3-hexanon-O-/2-D-[p-(p-jodphenoxy)phenoxy]-propionyl/-oxim; $\alpha_D^{20}$ = +50,64°, $n_D^{20}$ = 1,5652.

Beispiel 7

Zur Herstellung eines emulgierbaren Konzentrates werden die nachstehend aufgeführten Bestandteile dieses Konzentrates miteinander vermischt:

| | |
|---|---|
| Verbindung der Formel I (z.B. Endprodukt von Beispiel 1) | 500 g |
| Kondensationsprodukte eines Alkylphenoles und Aethylenoxyd; Dodecylbenzolsulfonsaures Calcium | 100 g |
| Epoxydiertes Soyaöl mit einem Oxiransauerstoffgehalt von ca. 6% | 25 g |
| Butyliertes Hydroxytoluol | 10 g |

Diese Mischung wird mit Xylol auf 1 Liter aufgefüllt.

## Patentansprüche

1. Verbindungen der allgemeinen Formel

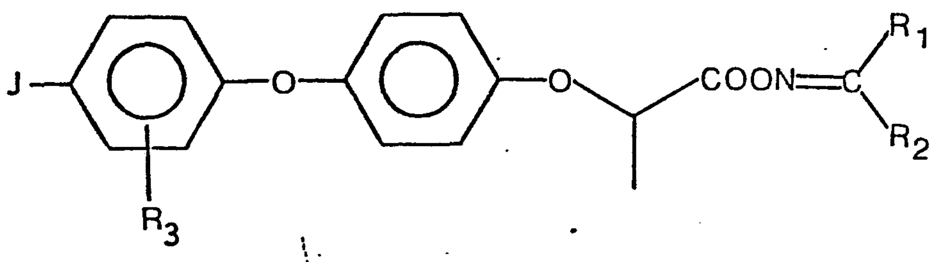

I

worin $R_1$ Wasserstoff, Alkyl mit 1-6 Kohlenstoffatomen oder Cycloalkyl mit 3-6 Kohlenstoffatomen, $R_2$ Wasserstoff, Alkyl mit 1-6 Kohlenstoffatomen, Alkenyl mit 2-6 Kohlenstoffatomen oder Alkinyl mit 2-6 Kohlenstoffatomen, oder $R_1$ und $R_2$ zusammen mit dem Kohlenstoffatom, an das sie geknüpft sind, einen Cyclopentan- oder Cyclohexanring bilden, der gegebenenfalls mit Alkyl mit 1-3 Kohlenstoffatomen mono-, di- oder trisubstituiert sein kann, und $R_3$ Wasserstoff, Halogen oder Nitro darstellt, mit der Massgabe, dass $R_1$ und $R_2$ nicht gleichzeitig Wasserstoff bedeuten.

2. Verbindungen der Formel I in Anspruch 1, worin $R_1$ und $R_2$ Alkylreste mit 1-2 Kohlenstoffatomen und $R_3$ Wasserstoff oder Chlor bedeuten.

3. Verbindungen der allgemeinen Formel I in Anspruch 1, worin $R_1$ und $R_2$ eine Methylgruppe und $R_3$ Wasserstoff oder Chlor bedeuten.

4. Aceton-O-/2-[p-(p-jodphenoxy)-phenoxy]propionyl/-oxim.

5. Cyclopropylmethylketon-O-/2-[p-(p-jodphenoxy)-phenoxy]propionyl/-oxim.

6. Aceton-O-/2-[p-(2-chlor-4-jodphenoxy)phenoxy]propionyl/-oxim.

7. Optisch aktive Verbindungen der D-Reihe entsprechend den Verbindungen in den Ansprüchen 1-6.

8. Eine Verbindung gemäss einem der Ansprüche 1-7 als Herbizid.

9. Unkrautbekämpfungsmittel, dadurch gekennzeichet, dass es mindestens eine Verbindung der allgemeinen Formel

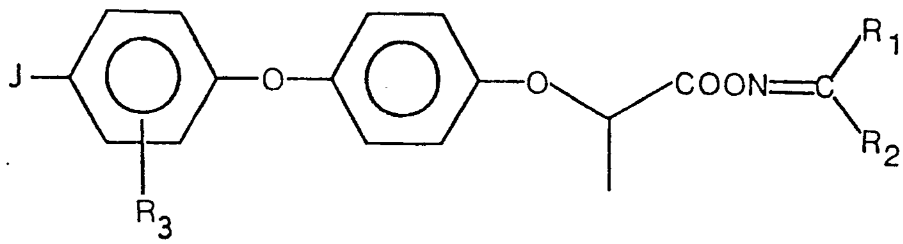

I

worin $R_1$ Wasserstoff, Alkyl mit 1-6 Kohlenstoffatomen oder Cycloalkyl mit 3-6 Kohlenstoffatomen, $R_2$ Wasserstoff, Alkyl mit 1-6 Kohlenstoffatomen, Alkenyl mit 2-6 Kohlenstoffatomen oder Alkinyl mit 2-6 Kohlenstoffatomen, oder $R_1$ und $R_2$ zusammen mit dem Kohlenstoffatom, an das sie geknüpft sind, einen Cyclopentan- oder Cyclohexanring bilden, der gegebenenfalls mit Alkyl mit 1-3 Kohlenstoffatomen mono-, di- oder trisubstituiert sein kann, und $R_3$ Wasserstoff, Halogen oder Nitro darstellt, mit der Massgabe, dass $R_1$ und $R_2$ nicht gleichzeitig Wasserstoff bedeuten,

und inertes Trägermaterial enthält.

10. Unkrautbekämpfungsmittel nach Anspruch 9, dadurch gekennzeichnet, dass es Aceton-O-/2-[p-(p-jodphenoxy)-phenoxy]propionyl/-oxim enthält.

11. Unkrautbekämpfungsmittel nach Anspruch 9, dadurch gekennzeichnet, dass es Aceton-O-/-2-[p-(2-chlor-4-jod-phenoxy)phenoxy]-propionyl/-oxim enthält.

12. Unkrautbekämpfungsmittel nach Anspruch 9, dadurch gekennzeichnet, dass es Cyclopropylmethylketon-O-/2-[p-(p-jodphenoxy)-phenoxy]-propionyl/-oxim enthält.

13. Unkrautbekämpfungsmittel nach einem der Ansprüche 9-12, dadurch gekennzeichnet, dass es das D-Isomere der Verbindung der Formel I enthält.

14. Verfahren zur Herstellung von Verbindungen der allgemeinen Formel

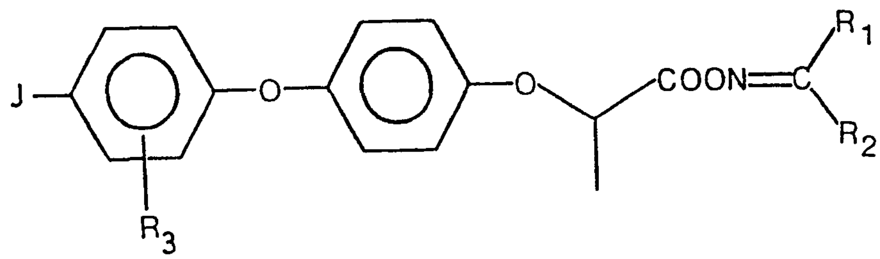

I

worin $R_1$ Wasserstoff, Alkyl mit 1-6 Kohlenstoffatomen oder Cycloalkyl mit 3-6 Kohlenstoffatomen, $R_2$ Wasserstoff, Alkyl mit 1-6 Kohlenstoffatomen, Alkenyl mit 2-6 Kohlenstoffatomen oder Alkinyl mit 2-6 Kohlenstoffatomen, oder $R_1$ und $R_2$ zusammen mit dem Kohlenstoffatom, an das sie geknüpft sind, einen Cyclopentan- oder Cyclohexanring bilden, der gegebenenfalls mit Alkyl mit 1-3 Kohlenstoffatomen mono-, di- oder trisubstituiert sein kann, und $R_3$ Wasserstoff, Halogen oder Nitro darstellt, mit der Massgabe, dass $R_1$ und $R_2$ nicht gleichzeitig Wasserstoff bedeuten,

dadurch gekennzeichnet dass man

a) eine Säure der Formel

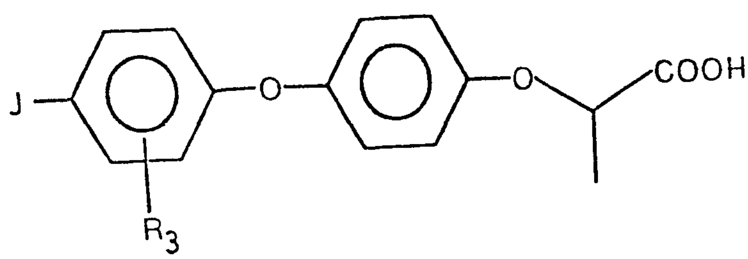

II

worin $R_3$ die für Formel I angegebene Bedeutung besitzt,

oder ein reaktionsfähiges Derivat einer solchen Säure, mit einem Oxim

$R_1R_2CNOH$ III

worin $R_1$ und $R_2$ die für Formel I angegebene Bedeutung besitzen,

umsetzt, oder

b) eine Verbindung der allgemeinen Formel

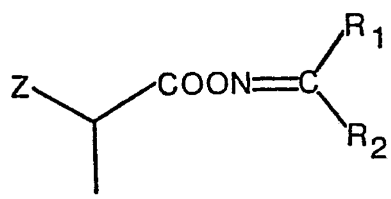

IV

worin Z Chlor, Brom, Jod, Mesyloxy oder Tosylosy bedeutet und $R_1$ und $R_2$ die für Formel I angegebene Bedeutung besitzen,

mit einer Verbindung der allgemeinen Formel

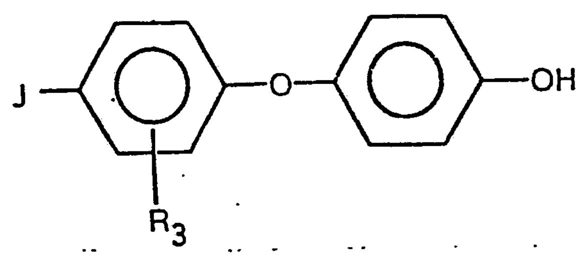

V

worin $R_3$ die oben angegebene Bedeutung hat,

oder einem Alkalimetallsalz davon, erforderlichenfalls in Gegenwart einer Base, umsetzt.

15. Verfahren nach Anspruch 14 zur Herstellung von Aceton-O-/2-[p-(p-jodphenoxy)phenoxy]propionyl/-oxim, dadurch gekennzeichnet, dass man (p-Jodphenoxy)phenol mit 2-Brompropionylacetonoxim umsetzt.

16. Verfahren nach Anspruch 14 zur Herstellung von Aceton-O-/2-[p-(p-jodphenoxy)phenoxy]propionyl/-oxim, dadurch gekennzeichnet, dass man 2-[p-(p-Jodphenoxy)-phenoxy]propionsäure in Gegenwart von Dicyclohexylcarbodiimid mit Acetonoxim umsetzt.

17. Verfahren nach Anspruch 14 zur Herstellung von Aceton-O-/2-[p-(2-chlor-4-jodphenoxy)phenoxy]propionyl/-oxim, dadurch gekennzeichnet, dass man 2-[p-(2-Chlor-4-jodphenoxy)phenoxy]propionsäure in Gegenwart von Dicyclohexylcarbodiimid mit Acetonoxim umsetzt.

18. Verfahren nach einem der Ansprüche 14-17, dadurch gekennzeichnet, dass man Ausgangsmaterialien verwendet, welche zum D-Isomeren der Verbindung der Formel I führen.

19. Verfahren zur Bekämpfung von Unkräutern, dadurch gekennzeichnet, dass man diese mit einem in den Ansprüchen 9-13 genannten Mittel behandelt.

20. Verwendung der in den Ansprüchen 1-7 genannten Verbindungen als Unkrautbekämpfungsmittel.

21. Verbindungen der allgemeinen Formel

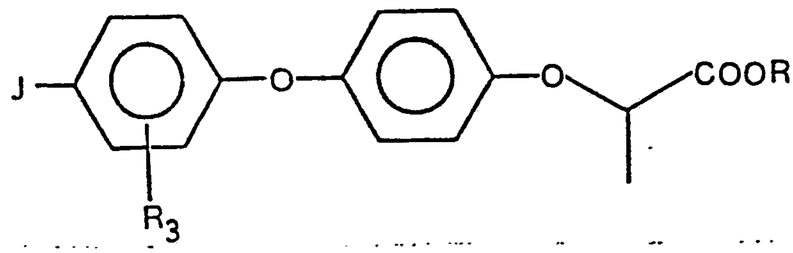

IIa

worin R Wasserstoff oder Alkyl mit 1-6 Kohlenstoffatomen und $R_3$ Wasserstoff, Halogen oder Nitro bedeuten. in D-Form.

22. 2-D-[p-(p-Jodphenoxy)phenoxy]-propionsäure.

23. 2-D-[p-(p-Jodphenoxy)phenoxy]-propionsäureäthylester.

24. 2-D-[p-(2-Chlor-4-jodphenoxy)phenoxy]-propionsäure.

25. 2-D-[p-(2-Chlor-4-jodphenoxy)phenoxy]-propionsäureäthylester.

***

Patentansprüche "für OESTERREICH"

1. Unkrautbekämpfungsmittel, dadurch gekennzeichnet, dass es mindestens eine Verbindung der allgemeinen Formel

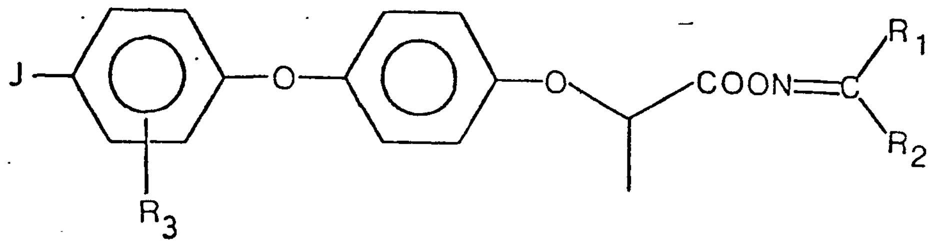

I

worin $R_1$ Wasserstoff, Alkyl mit 1-6 Kohlenstoffatomen oder Cycloalkyl mit 3-6 Kohlenstoffatomen, $R_2$ Wasserstoff, Alkyl mit 1-6 Kohlenstoffatomen, Alkenyl mit 2-6 Kohlenstoffatomen oder Alkinyl mit 2-6 Kohlenstoffatomen, oder $R_1$ und $R_2$ zusammen mit dem Kohlenstoffatom, an das sie geknüpft sind, einen Cyclopentan- oder Cyclohexanring bilden, der gegebenenfalls mit Alkyl mit 1-3 Kohlenstoffatomen mono-, di- oder trisubstituiert sein kann, und $R_3$ Wasserstoff, Halogen oder Nitro darstellt, mit der Massgabe, dass $R_1$ und $R_2$ nicht gleichzeitig Wasserstoff bedeuten,

und inertes Trägermaterial enthält.

2. Unkrautbekämpfungsmittel nach Anspruch 1, dadurch gekennzeichnet, dass es Aceton-O-/2-[p-(p-jodphenoxy)-phenoxy]propionyl/-oxim enthält.

3. Unkrautbekämpfungsmittel nach Anspruch 1, dadurch gekennzeichnet, dass es Aceton-O-/-2-[p-(2-chlor-4-jod-phenoxy)phenoxy]-propionyl/-oxim enthält.

4. Unkrautbekämpfungsmittel nach Anspruch 1, dadurch gekennzeichnet, dass es Cyclopropylmethylketon-O-/2-[p-(p-jodphenoxy)-phenoxy]-propionyl/-oxim enthält.

5. Unkrautbekämpfungsmittel nach einem der Ansprüche 1-4, dadurch gekennzeichnet, dass es das D-Isomere der Verbindung der Formel I enthält.

6. Verfahren zur Herstellung von Verbindungen der allgemeinen Formel

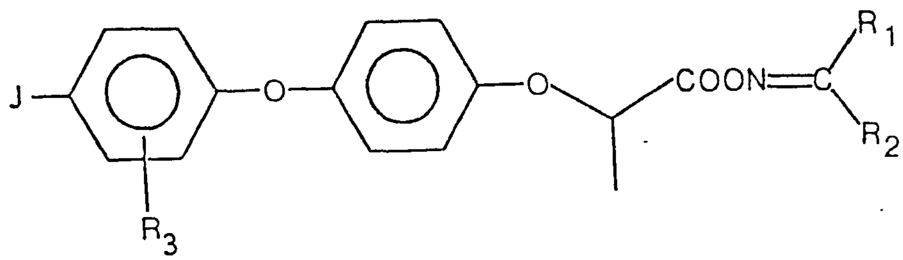

I

worin $R_1$ Wasserstoff, Alkyl mit 1-6 Kohlenstoffatomen oder Cycloalkyl mit 3-6 Kohlenstoffatomen, $R_2$ Wasserstoff, Alkyl mit 1-6 Kohlenstoffatomen, Alkenyl mit 2-6 Kohlenstoffatomen oder Alkinyl mit 2-6 Kohlenstoffatomen, oder $R_1$ und $R_2$ zusammen mit dem Kohlenstoffatom, an das sie geknüpft sind, einen Cyclopentan- oder Cyclohexanring bilden, der gegebenenfalls mit Alkyl mit 1-3 Kohlenstoffatomen mono-, di- oder trisubstituiert sein kann, und $R_3$ Wasserstoff, Halogen oder Nitro darstellt, mit der Massgabe, dass $R_1$ und $R_2$ nicht gleichzeitig Wasserstoff bedeuten,

dadurch gekennzeichnet dass man

a) eine Säure der Formel

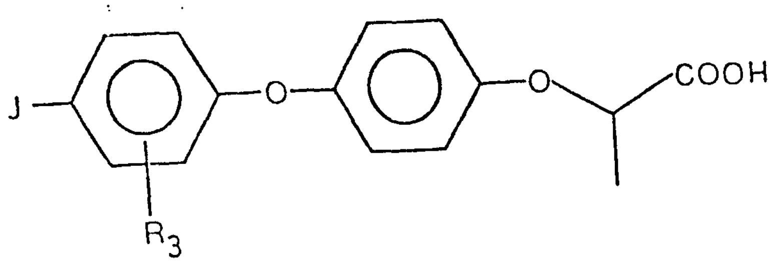

II

worin $R_3$ die für Formel I angegebene Bedeutung besitzt,

oder ein reaktionsfähiges Derivat einer solchen Säure, mit einem Oxim

$R_1R_2CNOH$ III

worin $R_1$ und $R_2$ die für Formel I angegebene Bedeutung besitzen,

umsetzt, oder

b) eine Verbindung der allgemeinen Formel

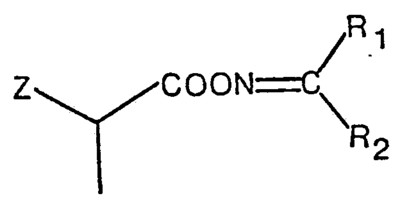

IV

worin Z Chlor, Brom, Jod, Mesyloxy oder Tosylosy bedeutet und $R_1$ und $R_2$ die für Formel I angegebene Bedeutung besitzen,

mit einer Verbindung der allgemeinen Formel

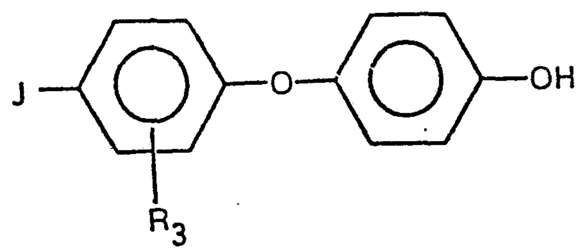

V

worin $R_3$ die oben angegebene Bedeutung hat,

oder einem Alkalimetallsalz davon, erforderlichenfalls in Gegenwart einer Base, umsetzt.

7. Verfahren nach Anspruch 6 zur Herstellung von Aceton-O-/2-[p-(p-jodphenoxy)phenoxy]propionyl/-oxim, dadurch gekennzeichnet, dass man (p-Jodphenoxy)phenol mit 2-Brompropionylacetonoxim umsetzt.

8. Verfahren nach Anspruch 6 zur Herstellung von Aceton-O-/2-[p-(p-jodphenoxy)phenoxy]propionyl/-oxim, dadurch gekennzeichnet, dass man 2-[p-(p-Jodphenoxy)-phenoxy]propionsäure in Gegenwart von Dicyclohexylcarbodiimid mit Acetonoxim umsetzt.

9. Verfahren nach Anspruch 6 zur Herstellung von Aceton-O-/2-[p-(2-chlor-4-jodphenoxy)phenoxy]propionyl/-oxim, dadurch gekennzeichnet, dass man 2-[p-(2-Chlor-4-jodphenoxy)phenoxy]propionsäure in Gegenwart von Dicyclohexylcarbodiimid mit Acetonoxim umsetzt.

10. Verfahren nach einem der Ansprüche 6-9, dadurch gekennzeichnet, dass man Ausgangsmaterialien verwendet, welche zum D-Isomeren der Verbindung der Formel I führen.

11. Verfahren zur Herstellung von Unkrautbekämpfungsmitteln, dadurch gekennzeichnet, dass man eine Verbindung der allgemeinen Formel

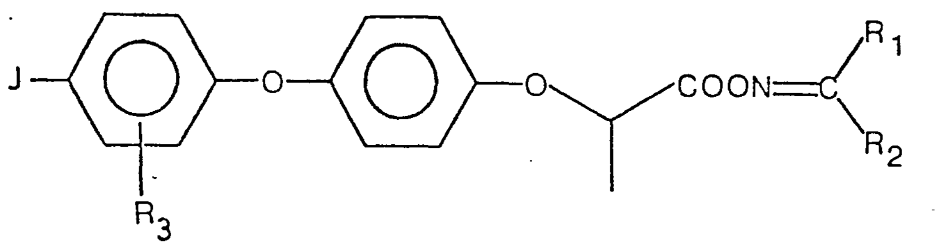

I

worin $R_1$ Wasserstoff, Alkyl mit 1-6 Kohlenstoffatomen oder Cycloalkyl mit 3-6 Kohlenstoffatomen, $R_2$ Wasserstoff, Alkyl mit 1-6 Kohlenstoffatomen, Alkenyl mit 2-6 Kohlenstoffatomen oder Alkinyl mit 2-6 Kohlenstoffatomen, oder $R_1$ und $R_2$ zusammen mit dem Kohlenstoffatom, an das sie geknüpft sind, einen Cyclopentan- oder Cyclohexanring bilden, der gegebenenfalls mit Alkyl mit 1-3 Kohlenstoffatomen mono-, di- oder trisubstituiert sein kann, und $R_3$ Wasserstoff, Halogen oder Nitro darstellt, mit der Massgabe, dass $R_1$ und $R_2$ nicht gleichzeitig Wasserstoff bedeuten,

unter Verwendung fester oder flüssiger Trägermaterialien und gegebenenfalls weiterer Hilfsmittel in eine für seine Applikation geeignete Form bringt.

12. Verfahren zur Bekämpfung von Unkräutern, dadurch gekennzeichnet, dass man diese mit einem in den Ansprüchen 1-5 genannten Mittel behandelt.

13. Verwendung der in den Ansprüchen 1-5 genannten Verbindungen als Unkrautbekämpfungsmittel.

***